# EUROPEAN PATENT APPLICATION

(11) **EP 1 920 751 A2**
(43) Date of publication of application: **14.05.2008**
(21) Application number: 07000133.4
(22) Date of filing: 04.01.2007
(51) Int. Cl.: A61J 7/00

(54) **Method of controlling compounding medicine in automatic medicine packaging machine**

(30) Priority: 13.11.2006 KR 20060111796
(71) Applicant: JVM Co., Ltd., Daegu 704-170 (KR)
(72) Inventor: Kim, Jun-ho, Dalseo-gu Daegu, 704-730 (KR)
(74) Representative: Grünecker, Kinkeldey, Stockmair & Schwanhäusser Anwaltssozietät

(57) **Abstract**

Disclosed is a medicine compounding control method of an automatic medicine packaging machine in which medicines having different medicine codes are easily compounded, the medicines are prevented from being disabled to be compounded due to discord between the medicine codes, and inconvenient use and delay of compounding of the medicines due to the different medicine codes are prevented. The method includes firstly determining whether there is a medicine having a name identical to reference medicines when the information is not identical; secondly determining whether the medicines quantities can be identical by changing an amount of the reference medicines when a medicine has an identical name; adjusting the amount of the reference medicines in the tablet cassette to correct the prescription data such that the quantity of the medicine having different medicine information can be identical to the reference medicines in the tablet cassette, when the medicine quantities can be identical.

## Description

### BACKGROUND OF THE INVENTION

### Field of the Invention

The present invention relates to an automatic medicine packaging machine, and more particularly, to a medicine compounding control method of an automatic medicine packaging machine in which medicines having medicine codes different from each other are easily compounded, the medicines are prevented from being disabled to be compounded due to discord between the medicine codes, and inconvenient use and delay of compounding of the medicines due to the discord between the medicine codes are prevented.

### Description of the Related Art

Generally, an automatic medicine packaging machine automatically packages medicine such as tablets dispensed by doses continuously.

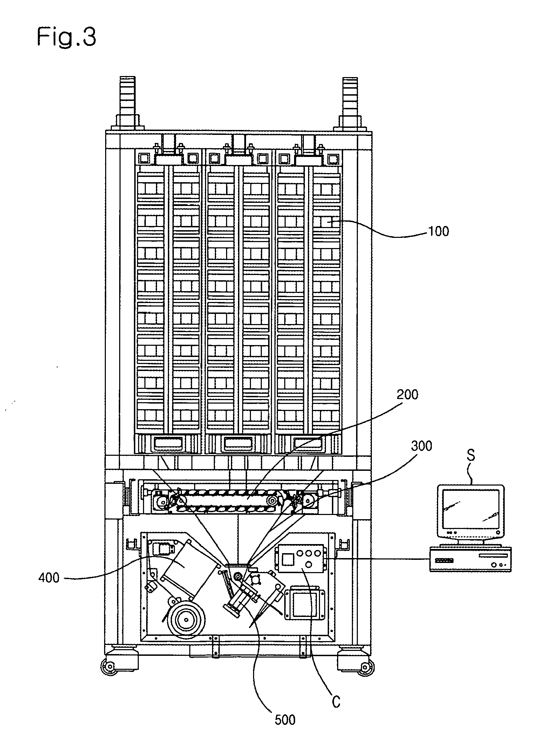

A conventional automatic medicine packaging machine is schematically illustrated in FIG. 3 and its configuration and operation will be described as follows.

As illustrated, the conventional automatic medicine packaging machine includes a plurality of tablet cassettes 100 provided on shelves that are disposed at the upper side of the machine, a manual tablet dispensing device 200 installed at the lower side, a hopper 300 provided at the lower sides of the tablet cassettes 100 and the manual tablet dispensing device 200, and a sealing device 500 to feed a packaging sheet printed by a printer 400 to seal the packaging sheet.

The tablet cassette 100, the printer 400, and the sealing device 500 are controlled by a controller C installed in the automatic medicine packaging machine. The controller C controls the tablet cassette 100, the printer 400, and the sealing device 500 according to prescription data inputted from a server computer S installed out of the automatic medicine packaging machine such that the medicines are compounded by a dose continuously, and the medicines discharged from the tablet cassette 100 according to the prescription are continuously packaged by a dose to form a series of medicine packets.

The manual dispensing device 200 is provided for a user to directly compound medicines that are not accommodated in the tablet cassette 100 and to package the same into packaging sheets.

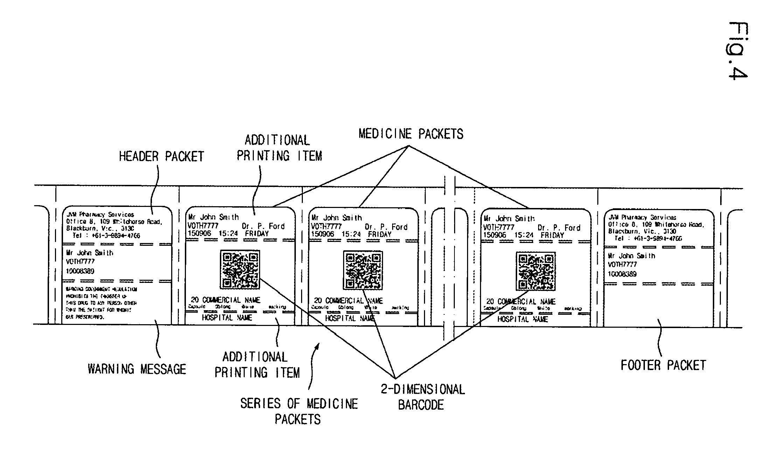

As illustrated in FIG. 4, in the series of medicine packets formed by the automatic medicine packaging machine, plural medicine packets in which the medicines are accommodated by a dose are connected to each other, and a header packet and a footer packer, which are empty and in which the medicines are not accommodated, are formed at the leading side and the ending side of the series of medicine packets.

A barcode is printed on front sides of the medicine packets and a patient's name, a hospital name, and a serial number of the patient, as additional printing items, are printed at upper and lower sides of the printed barcode.

Information about the patient and information about the medicines are printed on the header packet, and a warning message about the medicines is printed at a lower side of the printed information. Like the header packet, information about the patient and information about the packaged medicines are printed on the footer packet.

However, the conventional automatic medicine packaging machine has the following shortcomings.

When the inputted medicine code in prescription data is different from the medicine code of the medicine accommodated in the tablet cassette, that is, a reference medicine code preset in the controller, the conventional automatic medicine packaging machine cannot compound and package the medicines even when the medicines to be packaged are identical but their medicine codes are different from each other.

As such, since an additional setting operation to identify the medicine codes of the medicines is necessary when medicines are identical but their medicine codes are in discord with each other so that the medicines are not compounded, inconvenience is increased and overall compounding and packaging of the medicines are delayed.

Moreover, when the medicines are identical to each other but their quantities are different from each other so that the medicine information is in discord, the medicines cannot be automatically compounded. Thus, in this case, the medicines are manually compounded using the manual dispensing device so that it takes a lot of time and manpower to compound and package the medicines and the compounding of the medicines is delayed.

Further, the medicines are supplemented during the compounding and packaging when the medicines accommodated in the tablet cassette are completely consumed. However, when the supplement is disabled or it takes a lot of time to supplement the medicines, the compounding and packaging time must be delayed even when the medicines to be packaged are urgently packaged.

### SUMMARY OF THE INVENTION

Therefore, the present invention has been made in view of the above and/or other problems, and it is an aspect of the present invention to provide a compounding control method of an automatic medicine packaging machine in which medicines with different medicine codes are easily compounded, impossibility of compounding the medicines due to discord of the medicine codes is prevented, and inconvenience to use and a delay of compounding the medicines, caused by the discord of the medicine codes, are easily prevented.

It is another aspect of the present invention to provide a compounding control method of an automatic medicine packaging machine in which medicines with different medicine codes are displayed such that a user can inspect whether the medicine codes of the medicines are not identical to each other with the naked eye and a precise determination of the discord of the medicine codes is improved.

It is still another aspect of the present invention to provide a compounding control method of an automatic medicine packaging machine in which medicines having identical names and different quantities are identical to each other in the quantities such that the medicines with different quantities are easily prevented from being manually compounded and the compounding rate of the medicines is significantly increased due to reduction of the manual compounding.

It is still another aspect of the present invention to provide a compounding control method of an automatic medicine packaging machine in which medicines are compounded except for a medicine, which is disabled to be supplemented or which takes a lot of time to supplement, so that the compounding and packaging of the medicines are rapidly carried out and a delay of the compounding due to the supplementing of the medicine is easily prevented.

In accordance with the present invention, the above and other objects can be accomplished by the provision of a compounding control method of an automatic medicine packaging machine including: extracting medicine codes and medicine information of respective medicines to be compounded from prescription data inputted from a server computer; firstly comparing the extracted medicine codes with preset reference medicine codes to determine whether the extracted medicine codes are identical to the reference medicine codes; secondly comparing medicine information of a discord medicine code with medicine information of the preset reference medicine codes to determine whether the medicine information of a discord medicine code is identical to the medicine information of the preset reference medicine codes when the medicine code discord with the reference medicine codes is found as a result of the first comparison; and compounding respective medicines according to the prescription data by driving a tablet cassette when the medicine information is identical to each other according to the second comparison.

The compounding control method further includes displaying the extracted medicine codes having identical medicine information and the reference medicine codes, carried out after the second comparison.

The compounding control method further includes: firstly determining whether there is a medicine having a medicine name identical to those of reference medicines among the discord medicines when the medicine information is not identical as a result of the second comparison, carried out after the second comparison; secondly determining whether the quantities of the medicines can be identical to each other by changing an amount of the reference medicines when a medicine has an identical medicine name as a result of the first determination; adjusting the amount of the reference medicines accommodated in the tablet cassette to correct the prescription data such that the quantity of the medicine having the discord medicine information can be identical to the amount of the reference medicines accommodated in the tablet cassette, when the quantities of the medicines can be identical to each other.

The compounding control method further includes displaying the medicine information of the medicine, in which a medicine name and a quantity can be identical to those of the reference medicines, and the medicine information of the reference medicines on a display, carried out after adjusting the prescription data.

The compounding control method further includes displaying information of a medicine in which a medicine name is identical but a quantity is disabled to be identical such that the medicine, in which the quantity is disabled to be identical, is manually compounded, carried out after the second determination.

The compounding control method further includes determining whether to supplement the medicines or to continue the compounding of the medicines except for a medicine to be supplemented when a medicine supplement signal is inputted to the tablet cassette while driving the tablet cassette according to the prescription data, carried out after compounding the respective medicines.

The compounding control method further includes printing a medicine mane and a quantity of an excluded medicine on a packaging sheet when the excluded medicine is selected, carried out after the determining.

In the printing, the medicine name and the quantity of the excluded medicine are printed on an outer side of a medicine packet in which the excluded medicine must be packaged.

In the printing, the medicine name and the quantity of the excluded medicine are printed on an outer side of an empty medicine packet that is formed at a leading side of a series of medicine packets in which plural medicine packets to accommodate the excluded medicine are continuously connected to each other.

### BRIEF DESCRIPTION OF THE DRAWINGS

These and/or other objects and advantages of the present invention will become apparent and more readily appreciated from the following description of the embodiments, taken in conjunction with the accompanying drawings, in which:
FIG. 1 is a flowchart illustrating a compounding control method of an automatic medicine packaging machine according to the present invention;
FIG. 2 is a flowchart illustrating procedures of the compounding control method of an automatic medicine packaging machine according to the present invention;
FIG. 3 is a front sectional view schematically illustrating a conventional automatic medicine packaging machine; and
FIG. 4 is a plan view illustrating conventional medicine packets.

### DESCRIPTION OF THE PREFERRED EMBODIMENTS

Hereinafter, a compounding control method of an automatic medicine packaging machine according to embodiments of the present invention will be described in detail with reference to the accompanying drawings.

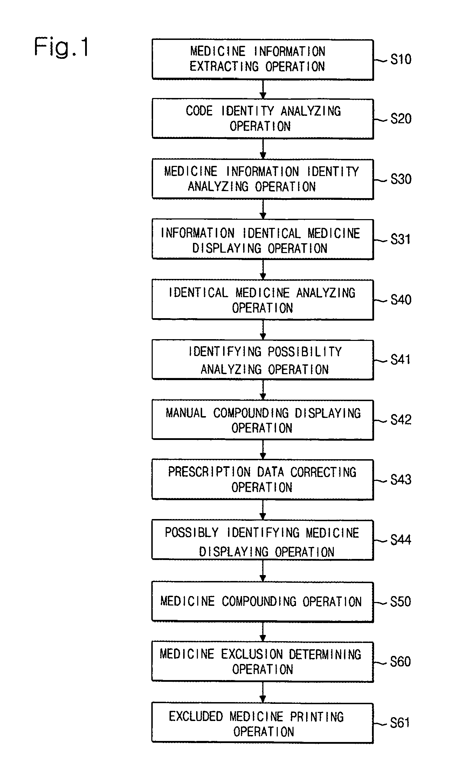

FIG. 1 is a flowchart illustrating a compounding control method of an automatic medicine packaging machine according to an embodiment of the present invention.

As illustrated, the compounding control method of an automatic medicine packaging machine includes a medicine information extracting operation (S10) of extracting medicine information from prescription data, a code identity analyzing operation (S20) of comparing medicine codes with each other to determine whether medicine codes are identical to each other, a medicine information identity analyzing operation (S30) of comparing medicine information with each other to determine whether the medicine information is identical to each other when the medicine codes are not identical to each other, and a medicine compounding operation (S50) of compounding the medicines when the medicine information is identical.

In the medicine information extracting operation (S10), respective medicine codes and information of medicines to be compounded are extracted from prescription data inputted from a server computer.

In this operation, a controller of the automatic medicine packaging machine, into which the prescription data is inputted from the server computer for the compounding and packaging of the medicines, extracts the medicine codes and the medicine information from the inputted prescription data. The medicine code means identification (ID) of a medicine assigned by a user, and the medicine information contains a medicine name, kind, a quantity, and the like.

In the code identity analyzing operation (S20), the medicine codes extracted in the medicine information extracting operation (S10) are compared with a preset reference medicine code to determine whether the medicine codes are identical to each other.

The reference medicine code means a medicine code assigned to a medicine accommodated in a tablet cassette of the automatic medicine packaging machine, and thus, a reference medicine means a medicine accommodated in the tablet cassette and reference medicine information means information about a medicine accommodated in the tablet cassette.

In the code identity analyzing operation (S20), the controller determines whether the medicine code extracted from the prescription data is identical to a medicine code of a medicine accommodated in the tablet cassette, namely, in the automatic medicine packaging machine.

When the medicine codes are identical to each other, since a medicine having medicine codes identical to the prescription data are in the tablet cassette, the medicines are compounded according to the prescription data. The compounding of the medicines means an operation of driving a tablet cassette in which medicines recorded in the prescription data are accommodated to discharge the medicines from the tablet cassette and to gather the discharged medicines. The gathered medicines are discharged down to be packaged into a packaging sheet.

In the medicine information identity analyzing operation (S30), when the extracted medicine code is not identical to the reference medicine code as a result of the analysis in the code identity analyzing operation (S20), medicine information of the different medicine code is compared with medicine information of the reference medicine code to determine whether the medicine information is identical to each other or not.

In this operation, a medicine with identical medicine information is found from the medicines with the different medicine codes to carry out the compounding of the medicines. In other words, when the different medicine codes are assigned by different hospitals or users but medicine information is identical to each other, in other words, medicines are identical to each other, the compounding of the medicines can be directly carried out without changing the medicine codes.

In the medicine compounding operation (S50), when the medicines with identical medicine information are found from the medicines whose medicine codes are determined different in the medicine information identity analyzing operation (S30), the medicines found can be compounded according to the prescription data by driving the tablet cassette.

The compounding control method of an automatic medicine packaging machine further includes an information identical medicine displaying operation (S31), carried out after the medicine information identity analyzing operation (S30), of displaying the extracted medicine codes having identical medicine information and the reference medicine codes on a display.

In the information identical medicine displaying operation (S31), the medicines having different medicine codes and identical medicine information are displayed such that the user can perceive the fact that the medicine codes are different from each other but the medicine information is identical to each other to easily carry out a post operation such as a correction of the medicine codes. Here, the display is preferably implemented by a monitor of the server computer or by a monitor installed in the automatic medicine packaging machine.

The compounding control method of an automatic medicine packaging machine further includes an identical medicine analyzing operation (S40) of determining whether there are medicines having names identical to each other from the medicines having different medicine information, an identifying possibility analyzing operation (S41) of determining whether there are medicines whose quantities are identical to each other, and a prescription data correcting operation (S43) of correcting the prescription data such that the quantities of the medicines are identical to each other when the identifying is possible.

In the identical medicine analyzing operation (S40), when the medicine information is different from each other according to the analysis of the medicine information after carrying out the medicine information identity analyzing operation (S30), whether there are medicines having names identical to those of the reference medicine among the different medicines is determined and analyzed.

In this operation, among the medicines having the medicine codes and the medicine information that are different from those of the reference medicines, the medicines having names identical to those of the reference medicines are analyzed and extracted. In other words, in this operation, since the medicine names are identical to each other but the quantities are different from each other, the medicines having medicine information different from that of the reference medicines are analyzed and extracted.

Here, the quantity of the medicine means a quantity of the medicine used by a dose.

For example, when the quantity of a medicine extracted from the prescription data is a single tablet of 400 mg aspirin as a dose but there is no 400 mg aspirin in the tablet cassette, whether there is aspirin among the reference medicines accommodated in the tablet cassette is analyzed.

In the identifying possibility analyzing operation (S41), when a medicine having a name identical to that of the reference medicine is found from the medicines having different medicine information as a result of the analysis in the identical medicine analyzing operation (S40), whether the quantity of the found medicine can be identical by changing the quantity of the reference medicine is determined and analyzed.

For example, if the medicine extracted from the prescription data is a 400 mg aspirin for a dose and the reference medicine, extracted by analyzing whether the medicine is identical or not, namely, a medicine accommodated in the tablet cassette is a 200 mg aspirin, two 200 mg aspirins correspond to a single 400 mg aspirin and thus it is determined that the quantity identification of the medicine is possible.

If the extracted reference medicine is a 300 mg aspirin per one pill, aspirin of 400 mg cannot be compounded by changing the amount of tablets discharged from the tablet cassette, the identification of the quantity of the medicines is determined impossible.

In the prescription data correcting operation (S43), when the identification of the quantity of medicines is possible in the identifying possibility analyzing operation (S41), the amount of the reference medicines discharged from the tablet cassette is adjusted to correct the prescription data such that the quantity of the reference medicines are identical to the quantity of the unidentified medicine.

For example, when the medicine extracted from the prescription data is a single pill of a 400 mg aspirin for a dose and the tablet cassette accommodates the reference medicine of a 200 mg aspirin, the prescription is corrected to identify the quantities of medicines such that two 200 mg aspirins are used to compound.

The compounding control method according to the embodiment of the present invention further includes a possibly identifying medicine displaying operation (S44), carried out after the prescription data correcting operation (S43), of displaying information of a medicine with an identical name and a quantity being able to be identified and information of the reference medicine.

In the possibly identifying medicine displaying operation (S44), information of a medicine whose quantity can be identical to that of the reference medicine to be compounded by correcting the prescription data and information of the reference medicine corresponding to the medicine are displayed such that the user perceives the fact and consequently it is prevented from determining the compounding is wrong because of the different quantities of the medicines while the packaged medicines are inspected with the naked eye.

The compounding control method according to the embodiment of the present invention further includes a manual compounding displaying operation (S42), carried out after the identifying possibility analyzing operation (S41), of displaying information of a medicine in which the identification is impossible such that the medicine having a quantity unable to be identified and an identical name is manually compounded.

In the manual compounding displaying operation (S42), since the medicine unable to be identified in the quantity cannot be compounded by driving the tablet cassette, the information of the medicine unable to be identified in the quantity is displayed such that the user perceives the information of the medicine unable to be identified and directly compounds the medicine using the manual dispensing device manually.

The compounding control method according to the embodiment of the present invention further includes a medicine exclusion determining operation (S60), carried out after the medicine compounding operation (S50), of determining whether medicine is supplemented or the compounding is continued by excluding the medicine when a medicine supplement signal is inputted while driving the tablet cassette according to the prescription data.

In the medicine exclusion determining operation (S60), when the supplement of medicines is impossible or it takes a lot of time for the supplement of medicines, the compounding of the medicines is carried out by excluding the medicine to be supplemented.

The compounding control method according to the embodiment of the present invention further includes an excluded medicine printing operation (S61), carried out after the medicine exclusion determining operation (S60), of printing a name and a quantity of the excluded medicine on the packaging sheet when the exclusion of the medicine is selected.

In the excluded medicine printing operation (S61), the excluded medicine is printed such that the user sees the printed information to easily add the excluded medicine later.

In the excluded medicine printing operation (S61), it is preferred that the name and the quantity of the excluded medicine are printed on the outer side of a corresponding medicine packet in which the excluded medicine must be packaged, or on the outer side of an empty medicine packet, which is formed at the leading side of a series of medicine packets that the corresponding medicine packets in which the excluded medicine must be packaged are continuously connected.

In other words, information of a medicine to be added is printed on the outer side of the medicine packet in which medicines are packaged by a dose or on the header packet such that the user easily and precisely adds the excluded medicine.

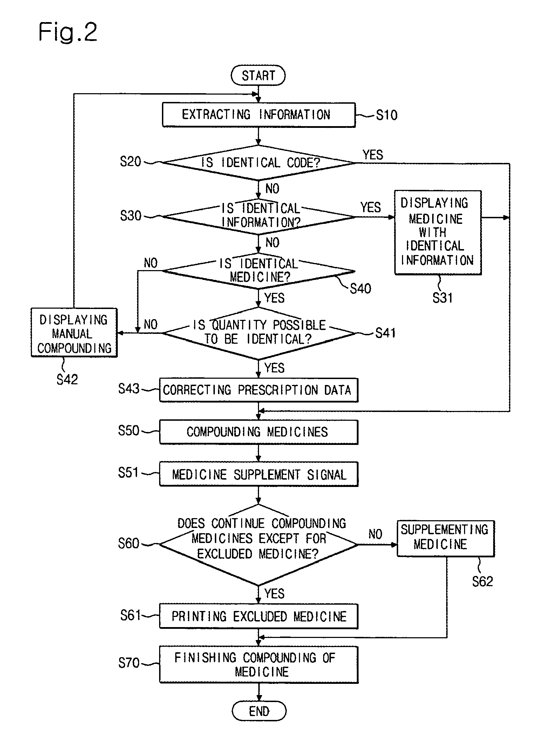

FIG. 2 is a flowchart illustrating procedures of the compounding control method of an automatic medicine packaging machine according to the present invention.

As illustrated, when the automatic medicine packaging machine is driven and the prescription data is inputted from the server computer to the controller, the controller extracts the medicine code and information of the medicine recorded in the prescription data (S10).

The controller compares the medicine code of the extracted medicine with a preset medicine code of the reference medicine corresponding to the medicine accommodated in the tablet cassette and determines whether the medicine code of the extracted medicine, namely, the medicine code of the prescription data is identical to the medicine code of the reference medicine accommodated in the tablet cassette (S20).

As a result of the comparison carried out by the controller, when the medicine code is identical to the medicine code of the reference medicine, the compounding of the medicines is directly carried out according to the prescription data (S50).

However, when the medicine codes are not identical to each other as a result of the comparison carried out by the controller, the controller determines whether the medicine code of the extracted prescription data is identical to the medicine code of the reference medicine accommodated in the tablet cassette (S30).

Since medicines, in which the medicines are determined identical when the medicine codes are not identical to each other but the medicine information is identical are identical to each other, a list of the medicines is displayed on the display such that the user perceives the fact (S31) and the medicine compounding operation is started to compound the medicines according to the prescription data (S50).

When both of the medicine codes and the medicine information is not identical as a result of the comparison carried out by the controller, whether there is a medicine having an identical medicine name in the medicine information or not is determined (S40).

When a medicine having an identical medicine name is not found as a result of the comparison carried out by the controller, since the medicine extracted from the prescription data cannot be automatically compounded using the tablet cassette, the extracted medicine is displayed as a medicine to be manually compounded such that the user manually compounds the medicines using the manual dispensing device (S42), and the starting operation is carried out again to receive another prescription data.

When a medicine in which the medicine code and the medicine information are not identical is found or a medicine name of the medicine extracted from the prescription data identical to the medicine name of the medicine accommodated in the tablet cassette is found as a result of the comparison carried out by the controller, whether the found medicine can be identical to the medicine extracted from the prescription data in the quantity is determined (S41).

When the quantities can be identical to each other according to the determination of the controller, since the medicine extracted from the prescription data cannot be automatically compounded using the tablet cassette, the extracted medicine is displayed as a medicine to be manually compounded such that the user manually compounds the extracted medicine (S42), and the starting operation is carried out again to receive another prescription data.

When the quantities of the medicines can be identical as a result of the determination of the controller, the prescription data is changed to adjust the amount of the medicines discharged from the tablet cassette such that the amount of the discharged medicine is identical to the quantity of the medicine extracted from the prescription data (S43), and the medicine compounding operation is directly carried out to compound the medicines according to the prescription (S50).

When medicines accommodated in the tablet cassette are completely discharged while driving the tablet cassette to compound the medicines according to the corrected prescription data, the medicine supplement signal is inputted to the controller (S51).

At that time, the controller stops the tablet cassette and determines whether the compounding of the medicines must be continued except for the medicines to be supplemented or not (S60). This determination may be carried out by presetting the controller to exclude a specific medicine when the medicine supplement signal is inputted or by directly inputting a medicine exclusion signal by the user.

When the medicines must be supplemented according to the determination of the controller, the controller is on standby while the medicines are supplemented by the user (S62) and then carries out and completes the compounding when the supplement of the medicines is completed (S70).

When the medicines to be supplemented are excluded according to the determination of the controller and the compounding is directly carried out, the list of the excluded medicines is printed (S61) and the compounding is carried out by excluding the medicines to finish the compounding of the medicines (S70).

As described above, the compounding of the identical medicines having different medicine codes is easily carried out, the impossibility of the compounding due to the discord between the medicine codes is prevented in advance, inconvenient use due to the discord between the medicine codes is settled, and the delay of the compounding is prevented.

Moreover, according to the present invention, the medicines with different medicine codes are displayed such that the user can inspect whether the medicine codes of the medicines are in discord with each other with the naked eye and a precise determination of the discord of the medicine codes is improved.

According to the present invention, the medicines having identical names and different quantities are identical to each other in the quantities such that the medicines with different quantities are easily prevented from being manually compounded and the compounding rate of the medicines is significantly increased due to reduction of the manual compounding.

Further, according to the present invention, the medicines are compounded except for a medicine, which is disabled to be supplemented or which takes a lot of time to supplement, so that the compounding and packaging of the medicines are rapidly carried out and a delay of the compounding due to the supplement of the medicine is easily prevented.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. A compounding control method of an automatic medicine packaging machine comprising:
extracting medicine codes and medicine information of respective medicines to be compounded from prescription data inputted from a server computer;
firstly comparing the extracted medicine codes with preset reference medicine codes to determine whether the extracted medicine codes are identical to the reference medicine codes;
secondly comparing medicine information of a discord medicine code with medicine information of the preset reference medicine codes to determine whether the medicine information of a discord medicine code is identical to the medicine information of the preset reference medicine codes when the medicine code discord with the reference medicine codes is found as a result of the first comparison; and
compounding respective medicines according to the prescription data by driving a tablet cassette when the medicine information is identical to each other according to the second comparison.

2. The compounding control method of an automatic medicine packaging machine according to claim 1, further comprising displaying the extracted medicine codes having identical medicine information and the reference medicine codes, carried out after the second comparison.

3. The compounding control method of an automatic medicine packaging machine according to claim 1, further comprising:
firstly determining whether there is a medicine having a medicine name identical to those of reference medicines among the discord medicines when the medicine information is not identical as a result of the second comparison, carried out after the second comparison;
secondly determining whether the quantities of the medicines can be identical to each other by changing an amount of the reference medicines when a medicine has an identical medicine name as a result of the first determination;
adjusting the amount of the reference medicines accommodated in the tablet cassette to correct the prescription data such that the quantity of the medicine having the discord medicine information can be identical to the amount of the reference medicines accommodated in the tablet cassette, when the quantities of the medicines can be identical to each other.

4. The compounding control method of an automatic medicine packaging machine according to claim 3, further comprising displaying the medicine information of the medicine, in which a medicine name and a quantity can be identical to those of the reference medicines, and the medicine information of the reference medicines on a display, carried out after adjusting the prescription data.

5. The compounding control method of an automatic medicine packaging machine according to claim 3, further comprising displaying information of a medicine in which a medicine name is identical but a quantity is disabled to be identical such that the medicine, in which the quantity is disabled to be identical, is manually compounded, carried out after the second determination.

6. The compounding control method of an automatic medicine packaging machine according to claim 1, further comprising determining whether to supplement the medicines or to continue the compounding of the medicines except for a medicine to be supplemented when a medicine supplement signal is inputted to the tablet cassette while driving the tablet cassette according to the prescription data, carried out after compounding the respective medicines.

7. The compounding control method of an automatic medicine packaging machine according to claim 6, further comprising printing a medicine mane and a quantity of an excluded medicine on a packaging sheet when the excluded medicine is selected, carried out after the determining.

8. The compounding control method of an automatic medicine packaging machine according to claim 7, wherein, in the printing, the medicine name and the quantity of the excluded medicine are printed on an outer side of a medicine packet in which the excluded medicine must be packaged.

9. The compounding control method of an automatic medicine packaging machine according to claim 7, wherein, in the printing, the medicine name and the quantity of the excluded medicine are printed on an outer side of an empty medicine packet that is formed at a leading side of a series of medicine packets in which plural medicine packets to accommodate the excluded medicine are continuously connected to each other.
